# EUROPEAN PATENT APPLICATION

(11) **EP 4 455 653 A1**
(43) Date of publication of application: **30.10.2024**
(21) Application number: 22910779.2
(22) Date of filing: 29.11.2022
(51) Int. Cl.: G01N 23/2251, G01N 21/17

(54) **PHYSICAL PROPERTY VALUE PREDICTING METHOD, AND PHYSICAL PROPERTY VALUE PREDICTING SYSTEM**

(30) Priority: 21.12.2021 JP 2021206742
(71) Applicant: MEC COMPANY., LTD., Amagasaki-shi, Hyogo 660-0822 (JP)
(72) Inventor: AKAGI, Masako, Amagasaki-shi, Hyogo 660-0822 (JP); HASCOET, Tristan, Kobe-shi, Hyogo 657-8501 (JP); DENG, Xuejiao, Kobe-shi, Hyogo 657-8501 (JP)
(74) Representative: TBK
(86) International application number: PCT/JP2022/043940
(87) International publication number: WO 2023/120061

(57) **Abstract**

The physical property value prediction method include: acquiring a prediction target image G1 of a prediction target material; inputting the prediction target image G1 into a prediction model machine-learned so as to receive an input of the image of the material as an explanatory variable and output the physical property value (Y) of the material, and outputting the physical property value (Y) of the material appearing in the prediction target image G1 as a predicted value.

## Description

### TECHNICAL FIELD

The present disclosure relates to a physical property value prediction method and a physical property value prediction system using machine learning.

### BACKGROUND ART

An electronic substrate has a laminated structure of a metal such as copper and a resin, and the quality of the electronic substrate corresponds to the adhesion between the metal and the resin. It is possible to evaluate a shape of a roughened metal surface subjected to surface treatment with a chemical to evaluate the adhesion of the metal of the electronic substrate. One of the evaluation items is, for example, measuring peel strength, but involving significant effort.

For example, Patent Document 1 discloses estimating a physical property value of a rubber material using machine learning.

### PRIOR ART DOCUMENT

### PATENT DOCUMENT

Patent Document 1: JP-A-2021-60457

### SUMMARY OF THE INVENTION

### PROBLEMS TO BE SOLVED BY THE INVENTION

In order to simplify the above evaluation, it would be desirable if the peel strength of the surface treated metal could be predicted by machine learning. It would be desirable if the physical property value of the material can be predicted by machine learning, not limited to the surface treated metal or peel strength.

The present disclosure provides a physical property value prediction method and a physical property value prediction system that can predicts a physical property value of a material using machine learning.

### MEANS FOR SOLVING THE PROBLEMS

According of the present disclosure, there is provided a physical property value prediction method comprising: acquiring a prediction target image of a prediction target material; and inputting the prediction target image into a prediction model machine-learned so as to receive an input of the image of the material as an explanatory variable and output the physical property value of the material, and outputting the physical property value of the material appearing in the prediction target image as a predicted value.

### BRIEF DESCRIPTION OF THE DRAWINGS

Fig. 1 is a block diagram illustrating a prediction system and a learning system of a first embodiment.
Fig. 2 is a diagram illustrating an example of an SEM image obtained by imaging a copper foil subjected to chemical treatment with an electron microscope.
Fig. 3 is a diagram obtained by plotting data of combinations of predicted values and measured values of Example 1.
Fig. 4 is a flowchart illustrating processing to be executed by the prediction system of the first embodiment.
Fig. 5 illustrates an example of an SEM image obtained by imaging a copper foil subjected to chemical treatment with an electron microscope.
Fig. 6 is illustrates an image of the feature map obtained using UNet based on the SEM image shown in Fig. 5.

### MODE FOR CARRYING OUT THE INVENTION

### <First embodiment>

Hereinafter, a first embodiment of the present disclosure will be described with reference to the drawings.

### [Learning system, prediction system]

A prediction system 2 (device) of the first embodiment predicts, using a prediction model 21, a physical property value (for example, peel strength) of a metal (copper) subjected to surface treatment with a chemical on the basis of an image obtained by imaging a surface of the metal. A learning system 1 (device) builds, using training data, the prediction model 21 on the basis of machine learning.

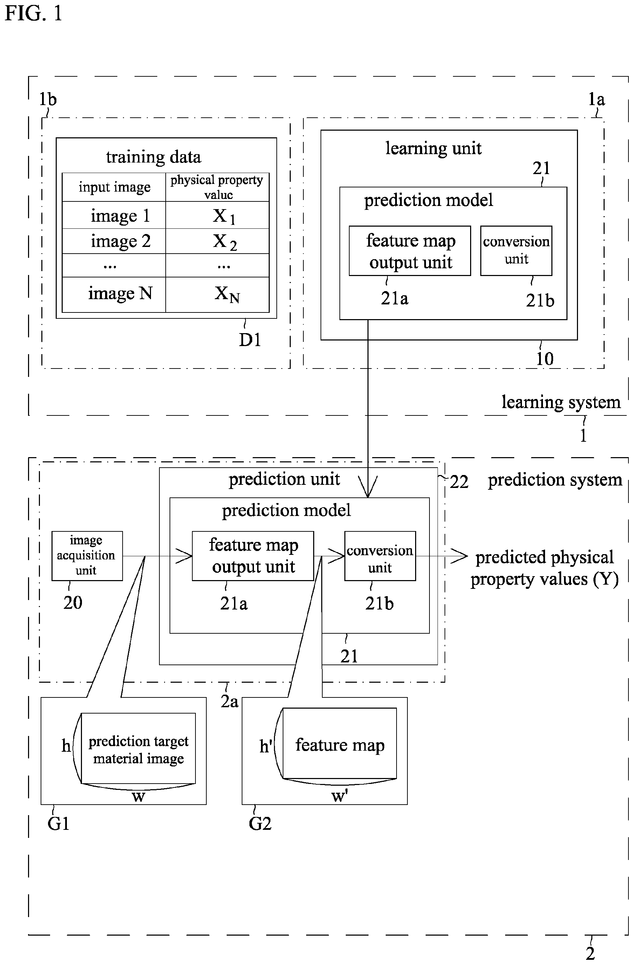

As illustrated in Fig. 1, the prediction system 2 includes an image acquisition unit 20 and a prediction unit 22. The learning system 1 includes training data D1 and a learning unit 10 that trains the prediction model 21. The training data D1 is stored in a memory 1b. The learning unit 10 is implemented by a processor 1a. The image acquisition unit 20 and the prediction unit 22 are implemented by a processor 2a. In the first embodiment, the processors 1a and 2a in one device implement each unit, but the present disclosure is not limited to such a configuration. For example, each processing may be distributed using a network, and a plurality of processors may each execute processing of the corresponding unit. That is, one or more processors execute processing.

The image acquisition unit 20 acquires an image G1 obtained by imaging a prediction target material. In the first embodiment, the image acquisition unit 20 acquires an SEM image (grayscale image) obtained by imaging a surface of a metal (copper) subjected to surface treatment with an electron microscope. The image G1 obtained by imaging the prediction target material is image data of vertical pixels h × horizontal pixels w.

The prediction model 21 is a model built, on the basis of machine learning, using the training data D1 in which images (for example, SEM images or camera images) obtained by imaging the material and physical property values (for example, peel strength) of the material are associated with each other so as to receive the input of an image as an explanatory variable and output the physical property value of a material. The learning unit 10 of the learning system 1 updates parameters of the prediction model 21 so to make a prediction result equal to the measured value of the training data. In Fig 1, in the training data D1, input images 1 to N (N represents the number of training images) and physical quantities (X₁, X₂, ..., X_{N}) that are measured values corresponding to the input images 1 to N are associated with each other.

Although various models can be used as the prediction model 21, in the first embodiment, the prediction model 21 includes a feature map output unit 21a that outputs a feature map G2 on the basis of an input image, and a conversion unit 21b that converts the feature map G2 into a physical property value (Y). In the first embodiment, UNet is used as the feature map output unit 21a. UNet is a U-shaped convolutional neural network (CNN)-based learning model. The conversion unit 21b uses global average pooling (GAP). The feature map G2 is data containing the number of pixels obtained by multiplying the number of pixels h' corresponding to the vertical pixels h of the input image G1 by the number of pixels w' corresponding to the horizontal pixels w of the input image, and each pixel has a feature of the physical property value of the material. The conversion unit 21b converts the feature map G2 into a single physical quantity (Y). Here, "corresponding to" covers a case where the feature map is an image identical in size to the original input image and a case where the feature map is an image different in size from the original input image but identical in aspect ratio, and means that it is possible to enlarge the feature map so as to make the feature map identical in size to the original input image.

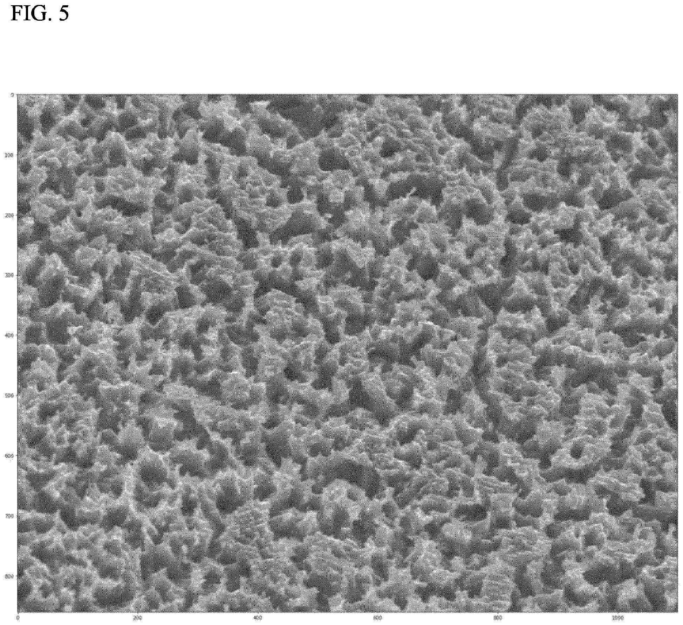

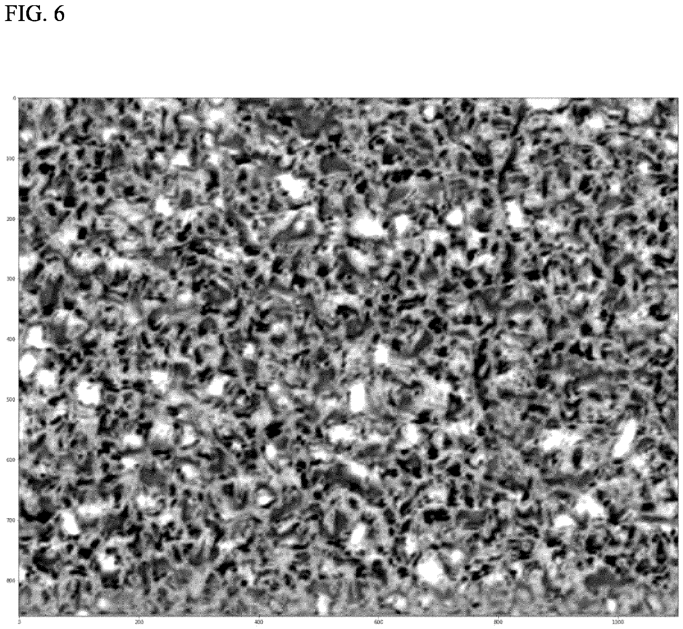

Fig. 5 illustrates an example of an SEM image obtained by imaging a copper foil subjected to chemical treatment with an electron microscope. Fig. 6 is illustrates an image of the feature map obtained using UNet based on the SEM image shown in Fig. 5. The image in the example shown in Fig. 6 is the same size as the input image shown in Fig. 5. Thus, the feature map has a number of features that corresponds to vertical pixels x horizontal pixels, each of the features indicates a feature related to the physical property values of the material.

### EXAMPLES

### [Example 1]

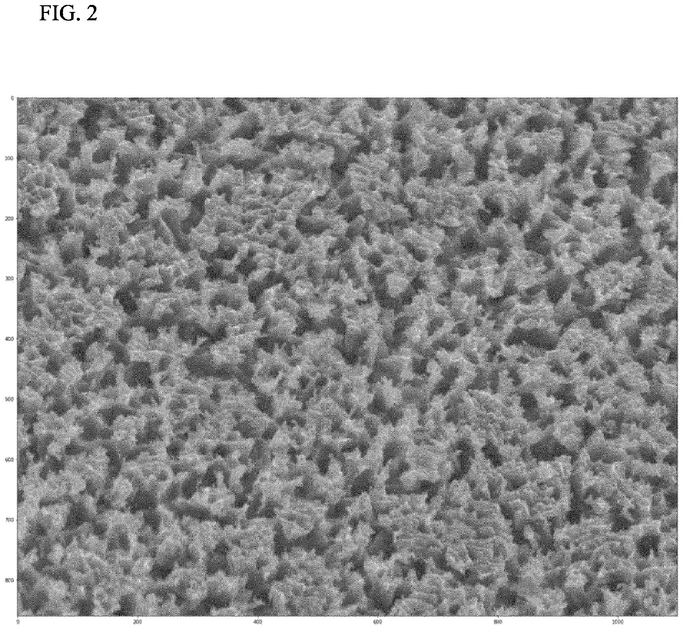

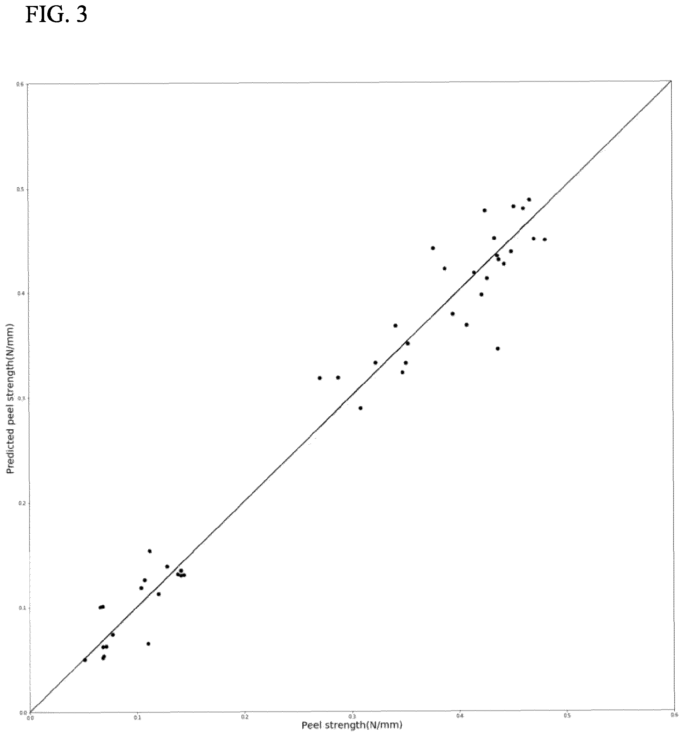

Example 1 is an example where the input image G1 is an SEM image obtained by imaging a copper foil subjected to chemical treatment with an electron microscope. Fig. 2 illustrates an example of the SEM image. The imaging conditions for the SEM image are a magnification of 3500x and a tilt angle of 45 degrees. The physical quantity was peel strength [N/mm]. This is an example where a copper surface is treated using a chemical A. 90 peel strengths were measured for 90 images. Half of the 90 pieces of data were used as data for training, and the remaining half were used as data for prediction. The mean squared error between the measured peel strengths and the predicted values was 0.0008. Fig. 3 is a diagram in which the horizontal axis represents measured peel strength and the vertical axis represents predicted peel strength, obtained by plotting data of combinations of predicted values and measured values.

### [Example 2]

In Example 2, the SEM image was used as the input image G1 as in Example 1. This is an example where a copper surface is treated using a chemical B. 72 peel strengths were measured for 72 images. Half of the 72 pieces of data were used as data for training, and the remaining half were used as data for prediction. The mean squared error between the measured peel strengths and the predicted values was 0.0012. The imaging conditions for the SEM image are the same as in Example 1.

### [Example 3]

In Example 3, the SEM image was used as the input image G1 as in Example 1. This is an example where a copper surface is treated using a chemical C. 39 peel strengths were measured for 39 images. Half of the 39 pieces of data were used as data for training, and the remaining half were used as data for prediction. The mean squared error between the measured peel strengths and the predicted values was 0.0021. The imaging conditions for the SEM image are the same as in Example 1.

### [Example 4]

Example 4 is an example where a plurality of physical property values are estimated from one SEM image that is the SEM image of Example 3. Specifically, setting the output of one UNet to a plurality of classes allows the same UNet to output (calculate) a plurality of physical property values. The plurality of physical property values each represent peel strength and roughness parameters (Sdr, Sdq). A first UNet for predicting peel strength, a second UNet for predicting Sdr, and a third UNet for predicting Sdq are parallelized. The mean squared error of peel strength was 0.001, the mean squared error of Sdr was 0.0003, and the mean squared error of Sdq was 0.0868.

### [Example 5]

Example 5 is an example where a copper surface is treated using a chemical D. The input image G1 is not the SEM image but a camera image captured by an optical camera. The camera image is subjected to only processing of separating a black background. RGB components contained in the camera image are separated into three monochrome images, and the three monochrome images are input into the prediction model 21. The physical property value was surface roughness Ra (arithmetic mean). 960 sets of data are used, half are used as training data, and the remaining half are used as prediction data. The mean squared error was 0.0153.

Note that it is also possible to convert a camera image containing RGB components into a grayscale image and input the intensity (lightness) of the grayscale image into the prediction model 21. Since there was no significant difference between the case of inputting the grayscale image and the case of inputting the three monochrome images of the RGB components, the three monochrome images of the RGB components were used in Example 5.

### [Example 6]

In Example 6, the camera image is used as the input image G1 as in Example 5. This is an example where a copper surface is treated using the chemical D. The physical property value was CIE 1976 lightness index L*. 320 sets of data are used, half are used as training data, and the remaining half are used as prediction data. The mean squared error was 11.05. L* adheres to JIS Z 8781-4.

### [Example 7]

In Example 7, the camera image is used as the input image G1 as in Example 5. This is an example where a copper surface is treated using the chemical D. The physical property value was a color coordinate a* in the CIE 1976 color space. 320 sets of data are used, half are used as training data, and the remaining half are used as prediction data. The mean squared error was 0.0062. a* adheres to JIS Z 8781-4.

### [Example 8]

In Example 8, the camera image is used as the input image G1 as in Example 5. This is an example where a copper surface is treated using the chemical D. The physical property value was a color coordinate b* in the CIE 1976 color space. 320 sets of data are used, half are used as training data, and the remaining half are used as prediction data. The mean squared error was 0.1294. b* adheres to JIS Z 8781-4.

### [Method for predicting physical property value of material]

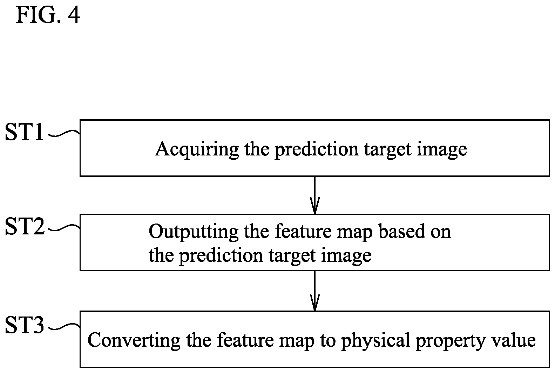

A method for predicting a physical property value of a material that is executed by the prediction system 2 will be described with reference to Fig. 4.

First, in step ST1, the image acquisition unit 20 acquires a prediction target image obtained by imaging a prediction target material. In the first embodiment, an SEM image or a camera image is acquired. In subsequent steps ST2 and ST3, the acquired prediction target image is input into the prediction model 21, and the physical property value of a material is output. Specifically, in step ST2, the feature map output unit 21a receives the input of the prediction target image and outputs the feature map G2. In step ST3, the conversion unit 21b converts the feature map G2 into the physical property value of the material.

### <Modification>

(1-1) In the embodiment illustrated in Fig. 1, the prediction model 21 includes the feature map output unit 21a that outputs the feature map G2, but may be a model that outputs a physical property value without outputting a feature map. For example, ResNet, which is a type of neural network, may be used. It goes without saying that any nonlinear model capable of processing images can be used.
(1-2) In the above-described embodiment, the prediction target material whose physical property value is to be predicted corresponds to a surface of a metal (copper) subjected to surface treatment by means of chemical reaction treatment with a chemical (etchant, polishing solution (chemical, electrolytic)) or the like, but is not limited to such a surface as long as the material has a uniform and fine surface shape. For example, the prediction target material may be a surface of a metal subjected to surface treatment by means of machining processing in which an external force such as polishing, rolling, or laser is applied. The prediction target material may be a surface coated with a coating material in which various pigments are dispersed and mixed. Further, in this case, the form (liquid, powder, etc.) of the coating material is not limited. The prediction target material may be a surface of a metal plated by electroplating, electroless plating, or the like. The prediction target material may be a surface of a film subjected to molding processing with an additive added and dispersed. The prediction target material may be a paper surface having a coated layer for receiving ink or a coated layer that provides other functionality. The material is not limited to a specific material, but may be a surface of a material subjected to calender molding, embossing molding, or the like.
(1-3) In the above-described embodiment, the peel strength has been given as the physical property value of a material, but the physical property value is not limited to the peel strength. For example, the physical property value of a material may be a physical property value related to adhesion, bonding, airtightness, water repellency, oil repellency, an antifouling property, a sliding property, a surface property (gloss, roughness), color tone, texture, a thermal property, an antibacterial property, or transmission loss.
(1-4) In the above-described embodiment, the metal surface image acquired by the SEM and the camera has been given as the material image, but the material image is not limited to such an image. Data that can be captured as an image, such as a waveform, a spectrum, a mapping image, and a numerical value, may also be provided as the material image. Examples of the data include a spectrum obtained by means of microspectroscopy (infrared, Raman, UV-Vis, etc.), energy dispersive X-ray spectroscopy (SEM-EDX), or ultrasonic testing used for non-destructive testing, and a mapping image using the spectrum.

As described above, the physical property value prediction method of the first embodiment may be executed by one or more processors, and may include: acquiring a prediction target image G1 of a prediction target material; inputting the prediction target image G1 into a prediction model machine-learned so as to receive an input of the image of the material as an explanatory variable and output the physical property value (Y) of the material, and outputting the physical property value (Y) of the material appearing in the prediction target image G1 as a predicted value.

As described above, since the physical property value of the material (metal) appearing in the prediction target image G1 can be predicted using the machine-learned prediction model 21, it is possible to reduce the monetary cost and the time cost as compared with a case where the physical property value is measured through a test or the like.

Although not particularly limited, as in the first embodiment, the prediction model 21 may include the feature map output unit 21a that outputs the feature map G2 with features related to physical property values related to the material on the basis of the input image G1, and the conversion unit 21b that converts the feature map G2 into a predicted value.

This allows the predicted value to be obtained from the feature map G2, which enables an explanation of the basis for the prediction made by the prediction model 21 to be given with the feature map G2.

Although not particularly limited, as in the first embodiment, the material to be imaged may be any one of a surface of a metal subjected to surface treatment, a surface coated with a coating material, a surface of a plated metal, a surface of a film, a paper surface, or a surface of a molded material. The above-described examples are preferred examples.

The system according to the first embodiment includes one or more processors that execute the above-described method.

The program according to the first embodiment is a program that causes one or more processors to execute the above-described method.

It is also possible to achieve the effects of the above-described method by executing such a program.

Although the embodiment of the present disclosure has been described above with reference to the drawings, it should be understood that specific configurations are not limited to the embodiment. The scope of the present disclosure is defined not only by the description of the above-described embodiment but also by the claims, and further includes equivalents of the claims and all modifications within the scope.

The structure employed in each of the above-described embodiments can be employed in any other embodiment. The specific configuration of each unit is not limited to the above-described embodiments, and various modifications can be made without departing from the gist of the present disclosure.

For example, the execution order of each processing such as operations, procedures, steps, and stages in the devices, systems, programs, and methods illustrated in the claims, the description, and the drawings may be any order unless the output of the previous processing is used in the subsequent processing. Even if the flows in the claims, the description, and the drawings are described using "first", "next", and the like for the sake of convenience, it does not mean that it is essential to execute in this order.

Each unit illustrated in Fig. 1 is implemented by a predetermined program executed by one or a plurality of processors, but each unit may include a dedicated memory or a dedicated circuit. In the system 1 (2) of the above-described embodiments, each unit is implemented by the single computer processor 1a (2a), but each unit may be distributed over and implemented by a plurality of computers or a cloud. That is, the above-described methods may be each executed by one or a plurality of processors.

The system 1 (2) includes the processor 1a (2a). For example, the processor 1a (2a) may be a central processing unit (CPU), a microprocessor, or other processing unit capable of executing computer-executable instructions. Further, the system 1 (2) includes the memory 1b (2b) for storing data of the system 1 (2). As an example, the memory 1b (2b) includes a computer storage medium, and includes a RAM, a ROM, an EEPROM, a flash memory or other memory technology, a CD-ROM, a DVD or other optical disc storage, a magnetic cassette, a magnetic tape, a magnetic disk storage or other magnetic storage device, or any other medium that can be used to store desired data and that can be accessed by the system 1.

### DESCRIPTION OF REFERENCE SIGNS

- G1: Prediction target image
- G2: Feature map
- 2: Prediction system
- 20: Image acquisition unit
- 21: Prediction model
- 21a: Feature map output unit
- 21b: Conversion unit
- 22: Prediction unit

## Claims

1. A physical property value prediction method comprising:
acquiring a prediction target image of a prediction target material; and
inputting the prediction target image into a prediction model machine-learned so as to receive an input of the image of the material as an explanatory variable and output the physical property value of the material, and outputting the physical property value of the material appearing in the prediction target image as a predicted value.

2. The physical property value prediction method according to claim 1, the prediction model include the feature map output unit that outputs the feature map on the basis of the input image, and the conversion unit that converts the feature map into a predicted value.

3. The physical property value prediction method according to claim 1 or 2, wherein the material to be imaged is any one of a surface of a metal subjected to surface treatment, a surface coated with a coating material, a surface of a plated metal, a surface of a film, a paper surface, or a surface of a molded material.

4. A physical property value prediction system comprising:
an image acquisition unit configured to acquires a prediction target image obtained by imaging a prediction target material; and
a prediction unit configured to input the prediction target image into a prediction model machine-learned so as to receive an input of the image of the material as an explanatory variable and output the physical property value of the material, and outputting the physical property value of the material appearing in the prediction target image as a predicted value.

5. The physical property value prediction system according to claim 4, the prediction model include the feature map output unit that outputs the feature map on the basis of the input image, and the conversion unit that converts the feature map into a predicted value.

6. The physical property value prediction system according to claim 4 or 5, wherein the material to be imaged is any one of a surface of a metal subjected to surface treatment, a surface coated with a coating material, a surface of a plated metal, a surface of a film, a paper surface, or a surface of a molded material.
